(19) Europäisches Patentamt European Patent Office Office européen des brevets

(11) **EP 4 006 670 B1**

(12) **EUROPEAN PATENT SPECIFICATION**

(45) Date of publication and mention
of the grant of the patent:
**05.08.2026 Bulletin 2026/32**

(21) Application number: **20844507.2**

(22) Date of filing: **09.06.2020**

(51) International Patent Classification (IPC):
**G05B 19/418** *(2006.01)*    **G05B 13/02** *(2006.01)*
**C21D 11/00** *(2006.01)*    **B21B 37/00** *(2006.01)*
**C21D 8/0221** *(2026.01)*    **C21D 9/46** *(2006.01)*

(52) Cooperative Patent Classification (CPC):
**G05B 13/0265; C21D 11/00; G05B 19/41875;**
B21B 37/00; C21D 8/0226; C21D 8/0236;
C21D 9/46; G05B 2219/32194; G05B 2219/45234;
G06N 20/20; Y02P 90/02

(86) International application number:
**PCT/JP2020/022637**

(87) International publication number:
**WO 2021/014804 (28.01.2021 Gazette 2021/04)**

(54) **METHOD FOR GENERATING QUALITY PREDICTION MODEL, QUALITY PREDICTION MODEL, QUALITY PREDICTION METHOD, METHOD FOR MANUFACTURING METAL MATERIAL, DEVICE FOR GENERATING QUALITY PREDICTION MODEL, AND QUALITY PREDICTION DEVICE**

VERFAHREN ZUR ERZEUGUNG EINES QUALITÄTSVORHERSAGEMODELLS, QUALITÄTSVORHERSAGEMODELL, QUALITÄTSVORHERSAGEVERFAHREN, VERFAHREN ZUR HERSTELLUNG EINES METALLMATERIALS, VORRICHTUNG ZUR ERZEUGUNG EINES QUALITÄTSVORHERSAGEMODELLS UND QUALITÄTSVORHERSAGEVORRICHTUNG

PROCÉDÉ DE PRODUCTION DE MODÈLE DE PRÉDICTION DE QUALITÉ, MODÈLE DE PRÉDICTION DE QUALITÉ, PROCÉDÉ DE PRÉDICTION DE QUALITÉ, PROCÉDÉ DE FABRICATION DE MATÉRIAU MÉTALLIQUE, DISPOSITIF DE PRODUCTION DE MODÈLE DE PRÉDICTION DE QUALITÉ ET DISPOSITIF DE PRÉDICTION DE QUALITÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.07.2019 JP 2019134701**

(43) Date of publication of application:
**01.06.2022 Bulletin 2022/22**

(73) Proprietor: **JFE Steel Corporation
Tokyo 100-0011 (JP)**

(72) Inventor: **SHIGEMORI, Hiroyasu
Tokyo 100-0011 (JP)**

(74) Representative: **Grünecker Patent- und
Rechtsanwälte
PartG mbB
Leopoldstraße 4
80802 München (DE)**

(56) References cited:
**WO-A1-2016/038705**    **JP-A- 2003 195 929**
**JP-A- 2012 137 813**    **JP-A- 2013 080 458**
**JP-A- 2019 074 969**

## Description

Field

The present invention relates to a quality prediction model generation method, a quality prediction model, a quality prediction method, a metal material manufacturing method, a quality prediction model generation device, and a quality prediction device.

Background

For methods of predicting quality with respect to certain requirement conditions, for example, there is a known method of calculating the distances between a plurality of past observed conditions stored in a record database and a desired requirement condition, calculating the weights of observed data (record data) based on the calculated distances, creating a function for fitting the neighborhood of the requirement condition based on the calculated weights, and predicting quality with respect to the requirement condition using the created function (refer to Patent Literature 1 to 8, for example).

A method of predicting quality is also present in published patent application JP 2019 074969.

Citation List

Patent Literature

Patent Literature 1: Japanese Patent Application Laid-open No. 2004-355189
Patent Literature 2: Japanese Patent Application Laid-open No. 2006-309709
Patent Literature 3: Japanese Patent Application Laid-open No. 2008-112288
Patent Literature 4: Japanese Patent Application Laid-open No. 2009-230412
Patent Literature 5: Japanese Patent Application Laid-open No. 2014-013560
Patent Literature 6: Japanese Patent Application Laid-open No. 2014-071858
Patent Literature 7: Japanese Patent Application Laid-open No. 2014-071859
Patent Literature 8: Japanese Patent Application Laid-open No. 2017-120638

Summary

Technical Problem

In the methods disclosed in Patent Literature 1 to 8, quality with respect to a certain requirement condition is calculated from data stored in a record database. This record database stores therein the record values of a plurality of manufacturing conditions and the record values of the quality of a metal material manufactured under these manufacturing conditions. As each of these manufacturing conditions and quality for one metal material, one representative value such as an average value is stored.

On the other hand, record values of manufacturing conditions for each process are collected in detail in the longitudinal direction of the metal material by sensors. However, in conventional techniques, the record values of manufacturing conditions collected in such detail have not been effectively utilized, which limits the improvement in prediction accuracy of the quality of metal materials.

The present invention has been made in consideration of the above, and it is an object of the present invention to provide a quality prediction model generation method, a quality prediction model, a quality prediction method, a metal material manufacturing method, a quality prediction model generation device, and a quality prediction device capable of predicting quality with respect to a certain manufacturing condition with high accuracy.

Solution to Problem

The invention is defined by the metal manufacturing method of claim 1. Optional features of the invention are provided in the dependent claims.

Advantageous Effects of Invention

According to the present invention, by generating a quality prediction model that associates a manufacturing condition of each process with the quality of a metal material manufactured under the manufacturing condition for each of predetermined areas, the quality of the metal material with respect to a certain manufacturing condition can be predicted more accurately than it is conventionally.

Brief Description of Drawings

FIG. 1 is a block diagram illustrating a configuration of a quality prediction model generation device and a quality prediction device according to an embodiment of the present invention.
FIG. 2 is a flowchart illustrating a flow of a quality prediction model generation method and a quality prediction method according to the embodiment of the present invention.
FIG. 3 is a chart illustrating an example of record data collected by a manufacturing record collection section 11 in the quality prediction model generation method according to the embodiment of the present invention.
FIG. 4 is a chart illustrating an example of record data edited by a manufacturing record edition section 12 in the quality prediction model generation method according to the embodiment of the present invention.
FIG. 5 is a diagram illustrating an example case in which metal materials are manufactured through a plurality of processes in the quality prediction model generation method according to the embodiment of the present invention.
FIG. 6 is a diagram illustrating an example of metal materials in each process in the quality prediction model generation method according to the embodiment of the present invention.
FIG. 7 is a chart illustrating an example of record data edited by an integrated process record edition section in the quality prediction model generation method according to the embodiment of the present invention.
FIG. 8 is a diagram schematically illustrating structures of a conventional record database and a record database of the present invention.
FIG. 9 includes charts illustrating prediction errors of a conventional method and a method of the present invention in predicting the tensile strength of a highly workable high-strength cold-rolled steel sheet.
FIG. 10 includes charts illustrating prediction errors of the conventional method and the method of the present invention in predicting the front and back surface hardness of a thick steel sheet.
FIG. 11 includes charts illustrating error ratios of the conventional method and the method of the present invention in predicting the front and back surface defects of a hot-dip galvanized steel sheet.

Description of Embodiments

A quality prediction model generation method, a quality prediction model, a quality prediction method, a metal material manufacturing method, a quality prediction model generation device, and a quality prediction device according to an embodiment of the present invention will be described with reference to the drawings.

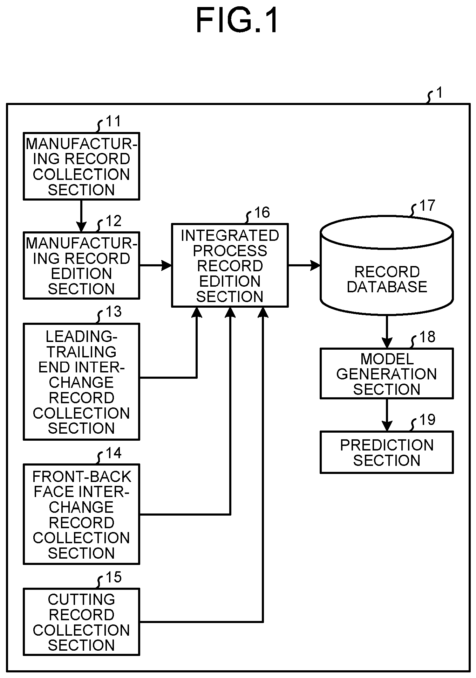

A configuration of the quality prediction device and the quality prediction model generation device according to the present embodiment will be explained with reference to FIG. 1. The quality prediction device is a device for predicting the quality of a metal material that is manufactured through one or more processes. Examples of a metal material in the present embodiment include a steel product, such as a semi-finished product such as a slab, and a product such as a steel sheet manufactured by rolling the slab.

A quality prediction device 1 is, specifically, implemented by a general-purpose information processing device such as a personal computer or a workstation, and includes main components such as a processor consisting of, for example, a central processing unit (CPU), and a memory (main memory) consisting of a random access memory (RAM), a read only memory (ROM), and the like.

As illustrated in FIG. 1, the quality prediction device 1 includes the manufacturing record collection section 11, the manufacturing record edition section 12, a leading-trailing end interchange record collection section 13, a front-back face interchange record collection section 14, a cutting record collection section 15, an integrated process record edition section 16, a record database 17, a model generation section 18, and a prediction section 19. Note that the quality prediction model generation device according to the present embodiment includes the elements of the quality prediction device 1 excluding the prediction section 19. The following also describes the quality prediction model generation device in the description of the quality prediction device 1.

A sensor, not illustrated in the drawings, is connected to the manufacturing record collection section 11. The manufacturing record collection section 11 collects a manufacturing record of each process according to the measurement cycle of the sensor, and outputs the record to the integrated process record edition section 16. The aforementioned "manufacturing record" includes a manufacturing condition of each process and the quality of a metal material manufactured through each process. Examples of the aforementioned "manufacturing condition" include the composition, temperature, pressure, plate thickness, and threading speed of the metal material in each process. In addition, examples of the aforementioned "quality of metal material" include tensile strength and defect contamination rate (the number of defects appearing per unit length).

A manufacturing condition of each process collected by the manufacturing record collection section 11 includes not only an actual measured value of the manufacturing condition measured by the sensor, but also a preset value of the manufacturing condition. In other words, no sensor may be installed in some processes. In such cases, a set value is collected as a manufacturing record instead of a record value.

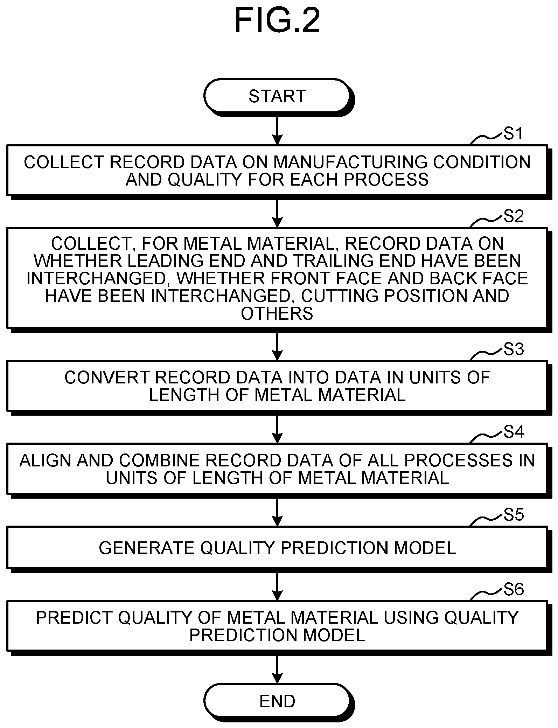

The manufacturing record collection section 11 collects a manufacturing condition of each process for each of predetermined areas of the metal material. In addition, the manufacturing record collection section 11 evaluates and collects the quality of the metal material manufactured through each process for each of the predetermined areas described above. The "predetermined area" mentioned above refers to a certain area in the longitudinal direction of a metal material when the metal material is a slab or steel sheet, for example. This predetermined area is determined based on the travel distance (threading speed) of the metal material based on the conveyance direction in each process. Specific processing by the manufacturing record collection section 11 will be described later (refer to FIG. 2).

In the configuration illustrated in FIG. 1, only one manufacturing record collection section 11 is provided, and the premise is that the data on the manufacturing record of each process (hereinafter referred to as "record data") is collected by this one manufacturing record collection section 11. For example, however, a plurality of manufacturing record collection sections 11 may be provided to match the number of processes, and the record data of the processes may be collected by the respective manufacturing record collection sections 11.

The manufacturing record edition section 12 edits the record data of each process input from the manufacturing record collection section 11. In other words, the manufacturing record edition section 12 edits the record data collected in units of time by the manufacturing record collection section 11 into record data in units of the length of the metal material, and outputs the data to the integrated process record edition section 16. Specific processing by the manufacturing record edition section 12 will be described later (refer to FIG. 2).

A material loading machine for loading the metal material in each process, which is not illustrated in the drawings, is connected to the leading-trailing end interchange record collection section 13. Through the material loading machine, the leading-trailing end interchange record collection section 13 collects record data on whether the leading end and the trailing end of the metal material have been interchanged (reversed) when the metal material from a previous process is loaded for a subsequent process. Then, the leading-trailing end interchange record collection section 13 outputs, to the integrated process record edition section 16, the record data on whether the leading end and the trailing end of the metal material have been interchanged.

The material loading machine described above is connected to the front-back face interchange record collection section 14. Through the material loading machine, the front-back face interchange record collection section 14 collects record data on whether the front face and the back face of the metal material have been interchanged (reversed) when the metal material from a previous process is loaded for a subsequent process. Then, the front-back face interchange record collection section 14 outputs, to the integrated process record edition section 16, the record data on whether the front face and the back face of the metal material have been interchanged.

A cutting machine for cutting the leading end portion and the trailing end portion of the metal material, which is not illustrated in the drawings, is connected to the cutting record collection section 15. Through the cutting machine, the cutting record collection section 15 collects record data such as a cutting position (distance from the leading end of the metal material at the time of cutting) and the number of cuts (hereinafter referred to as "cutting position and others") for each metal material. The cutting record collection section 15 then outputs the record data on the cutting position and others of the metal material to the integrated process record edition section 16.

As with the aforementioned manufacturing record collection section 11, only one of each of the leading-trailing end interchange record collection section 13, the front-back face interchange record collection section 14, and the cutting record collection section 15 may be provided, or a plurality of each of these sections may be provided to match the number of processes.

The integrated process record edition section 16 edits the record data input from the manufacturing record edition section 12, the leading-trailing end interchange record collection section 13, the front-back face interchange record collection section 14, and the cutting record collection section 15. The integrated process record edition section 16 stores, in the record database 17, the manufacturing condition of each process and the quality of the metal material manufactured under this manufacturing condition in association with each other for each of the predetermined areas.

The integrated process record edition section 16 identifies a predetermined area while taking into account, for every metal material in each process, whether the leading end and the trailing end have been interchanged, whether the front face and the back face have been interchanged, and the cutting position. The integrated process record edition section 16 then stores, in the record database 17, the manufacturing condition of each process and the quality of the metal material manufactured under this manufacturing condition in association with each other for each of the predetermined areas in a form that is able to distinguish, for the material in each process, whether the leading end and the trailing end have been interchanged, whether the front face and the back face have been interchanged, and the cutting position.

Furthermore, when each process is a rolling process, for example, and the shape of a metal material is deformed as it goes through each process, the integrated process record edition section 16 identifies a predetermined area by evaluating the volume of the metal material from the leading end, and stores, in the record database 17, the manufacturing condition of each process and the quality of the metal material manufactured under this manufacturing condition in association with each other for each of the predetermined areas. This record database 17 stores therein the record data edited by the integrated process record edition section 16.

The model generation section 18 generates a quality prediction model that predicts the quality of a metal material for each of predetermined areas based on the manufacturing condition for each of the predetermined areas in each process stored in the record database 17. The model generation section 18 uses, for example, XGBoost as a machine learning method. Various other methods of machine learning can be used, such as linear regression, local regression, principal component regression, PLS regression, a neural network, a regression tree, and a random forest.

The prediction section 19 uses the quality prediction model generated by the model generation section 18 to predict the quality of a metal material manufactured under a certain manufacturing condition for each of predetermined areas. For example, when the metal material to be predicted is a slab, while conventional methods predict the quality of the entire slab, the present embodiment can predict the quality of the slab for each of predetermined areas in the length direction.

A quality prediction method and a quality prediction model generation method according to the present embodiment will be explained with reference to FIG. 2 to FIG. 7. The quality prediction method according to the present embodiment performs the processing of step S1 to step S6 illustrated in FIG. 2. The quality prediction model generation method according to the present embodiment performs the processing of step S1 to step S5 excluding step S6 illustrated in FIG. 2.

First, the manufacturing record collection section 11 collects record data on a manufacturing condition and quality for each process (step S1). The manufacturing record collection section 11 collects the record data on the manufacturing condition and quality for each metal material and for each process.

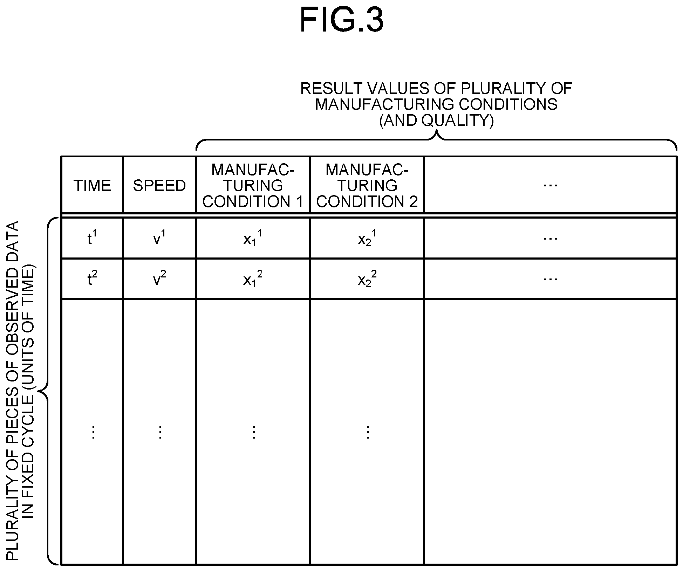

The record data collected by the manufacturing record collection section 11 is, for example, data in which the record values (or set values) of a plurality of manufacturing conditions are arranged by time, as illustrated in the table in FIG. 3. The record data illustrated in FIG. 3 includes times $t^1$, $t^2$..., the speeds of a metal material (threading speed) $v^1$, $v^2$..., and a plurality of manufacturing conditions $x_1^1$, $x_1^2$..., $x_2^1$, $x_2^2$... measured by sensors at the respective times. The record data collected in the final process of a plurality of processes includes an item related to the quality of the metal material in addition to the items illustrated in the FIG. 3.

Then, the leading-trailing end interchange record collection section 13, the front-back face interchange record collection section 14, and the cutting record collection section 15 collect record data on whether the leading end and the trailing end of the metal material have been interchanged in each process, whether the front face and the back face of the metal material have been interchanged in each process, and the cutting position and others of the metal material in each process (step S2).

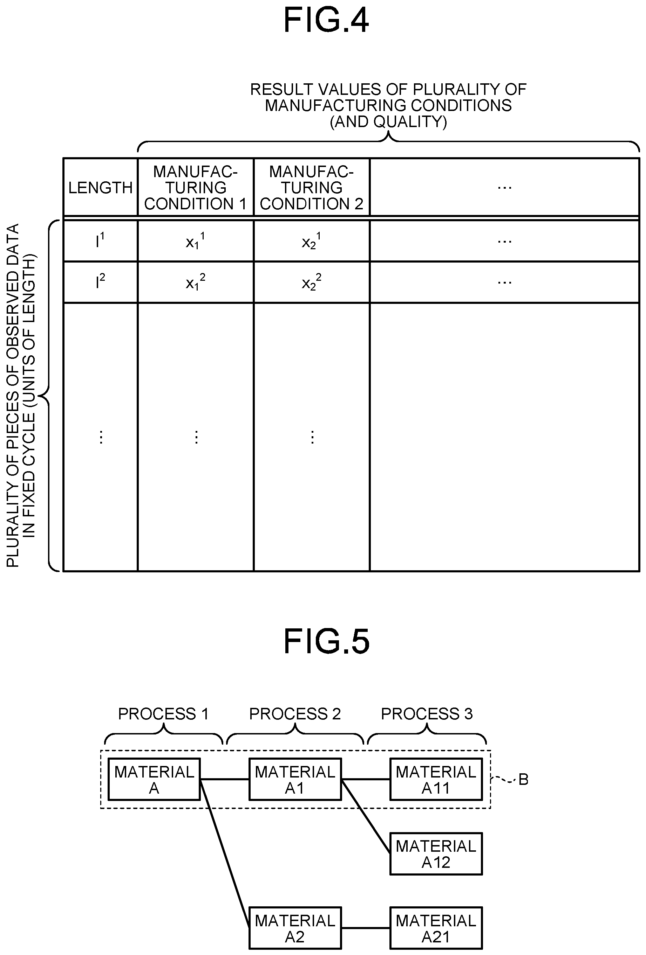

The manufacturing record edition section 12 then converts the record data collected by the manufacturing record collection section 11 into data in units of the length of the metal material (step S3). In other words, the manufacturing record edition section 12 converts the record data collected in units of time, as illustrated in FIG. 3, into record data in units of the length of the metal material, as illustrated in FIG. 4. The following describes a method of converting the record data in FIG. 3 into the record data in FIG. 4.

First, the manufacturing record edition section 12 calculates the position of the metal material at each time in FIG. 3, using the principle that multiplying time and speed (threading speed) records in distance. Next, the manufacturing record edition section 12 detects the leading end and the trailing end of the metal material using a function that record data is recorded when the metal material is passing by the sensors installed for each process, and missing values are recorded when the metal material is not passing. Next, the manufacturing record edition section 12 creates record data corresponding to positions of the metal material from the leading end to the trailing end, except for the time when the metal material is not passing by the sensor.

The data, as it is, is in units of the length of the metal material, but not in a fixed cycle. Thus, the data is converted into record data in units of the length of the metal material and in a fixed cycle by, for example, linear interpolation. In other words, in each process, when the threading speed of the metal material is slow, the record data that can be collected becomes finer, and when the threading speed of the metal material is fast, the record data that can be collected becomes coarser. Therefore, interpolation as described above is used to align the granularity of the record data. The manufacturing record edition section 12 performs the above processing to create the record data in units of the length of the metal material as illustrated in FIG. 4.

Then, the integrated process record edition section 16 combines the record data of all processes, aligning them in units of the length of the metal material (step S4). The integrated process record edition section 16 combines the record data of the manufacturing conditions and the quality of the metal material in all the processes while aligning them in units of the length of the metal material at the output side of the final process based on the record data in units of the length of the metal material created by the manufacturing record edition section 12, and the record data on whether the leading end and the trailing end of the metal material have been interchanged, whether the front face and the back face of the metal material have been interchanged, and the cutting position and others of the metal material collected by the leading-trailing end interchange record collection section 13, the front-back face interchange record collection section 14, and the cutting record collection section 15, respectively.

In this way, the integrated process record edition section 16 associates the manufacturing conditions of each process with the quality of the metal material manufactured under these manufacturing conditions for each of predetermined areas in the length direction of the metal material, and stores them in the record database 17. The following describes an example of processing by the integrated process record edition section 16.

For example, a case is considered in which a metal material (material) is manufactured through process 1, process 2, and process 3 as illustrated in FIG. 5. Processes 1 through 3 are rolling processes, for example, and the longitudinal length of the material increases with each process. As illustrated in FIG. 5, material A is divided into material A1 and material A2 when moving from process 1 to process 2, and material A1 is divided into material A11 and material A12 when moving from process 2 to process 3.

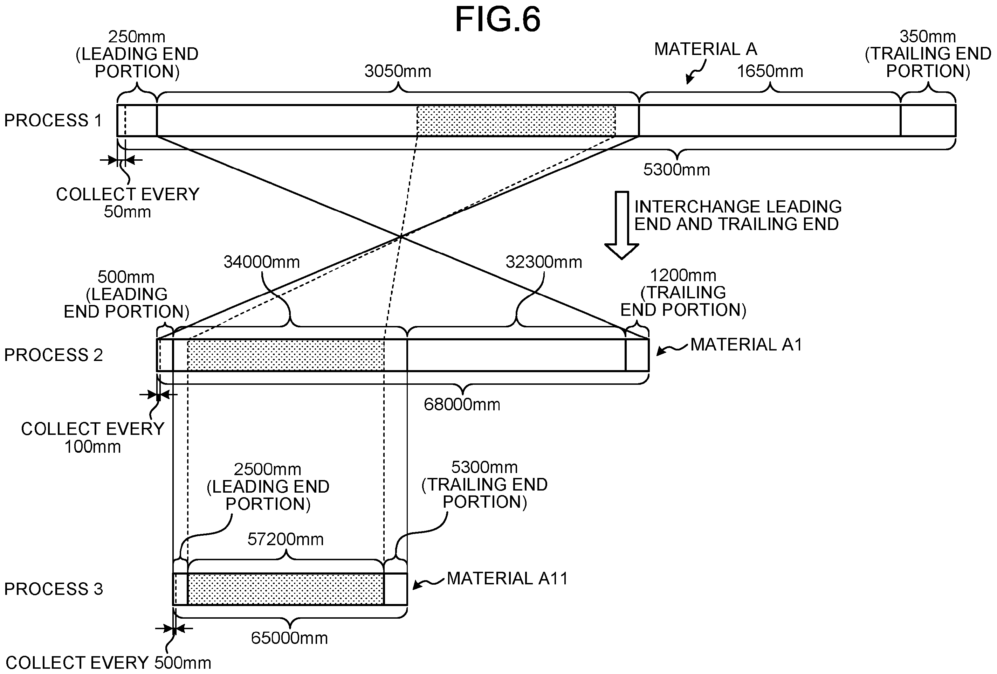

FIG. 6 illustrates an image of the materials in each process, focusing on part B in FIG. 5. For example, for material A in process 1, the manufacturing record collection section 11 collects record data for M1 items, for example, $X_1^1$ to $X_1^{M1}$, every 50 mm in an area of 5300 mm in length from the leading end to the trailing end. For material A, the cutting record collection section 15 collects record data indicating that the leading end portion from 0 mm (leading end) to 250 mm has been truncated, material A1 has been taken from 250 mm to 3300 mm, material A2 has been taken from 3300 mm to 4950 mm, and the trailing end portion from 4950 mm to 5300 mm (trailing end) has been truncated.

Then, for material A1 in process 2, the leading-trailing end interchange record collection section 13 collects the record data of "the leading and trailing end interchanged". For material A1, the manufacturing record collection section 11 collects record data for M2 items, for example, $X_2^1$ to $X_2^{M2}$, every 100 mm in an area of 68000 mm in length from the leading end to the trailing end. For material A1, the cutting record collection section 15 collects record data indicating that the leading end portion from 0 mm (leading end) to 500 mm has been truncated, material A11 has been taken from 500 mm to 34500 mm, material A12 has been taken from 34500 mm to 66800 mm, and the trailing end portion from 66800 mm to 68000 mm (trailing end) has been truncated.

Then, for material A11 in process 3, the leading-trailing end interchange record collection section 13 collects the record data of "the leading and trailing ends not interchanged". For material A11, the manufacturing record collection section 11 collects record data for M3 items, for example, $X_3^1$ to $X_3^{M3}$, every 500 mm in an area of 65000 mm in length from the leading end to the trailing end. For material A11, the cutting record collection section 15 collects record data indicating that the leading end portion from 0 mm (leading end) to 2500 mm has been truncated, material A11 has been taken from 2500 mm to 59700 mm, and the trailing end portion from 59700 mm to 65000 mm (trailing end) has been truncated.

In order to combine the record data of all processes, which are collected in detail in the longitudinal direction by sensors not illustrated in the drawings, in units of the length of the metal materials in the final process, while taking into account, for every metal material in each process, the record data on whether the leading end and the trailing end have been interchanged, whether the front face and the back face have been interchanged, and the cutting position and others, the integrated process record edition section 16 scales the material lengths in process 2 and process 1 to match the material length in process 3, the final process, as illustrated in FIG. 5 (refer to the dashed line in FIG. 5).

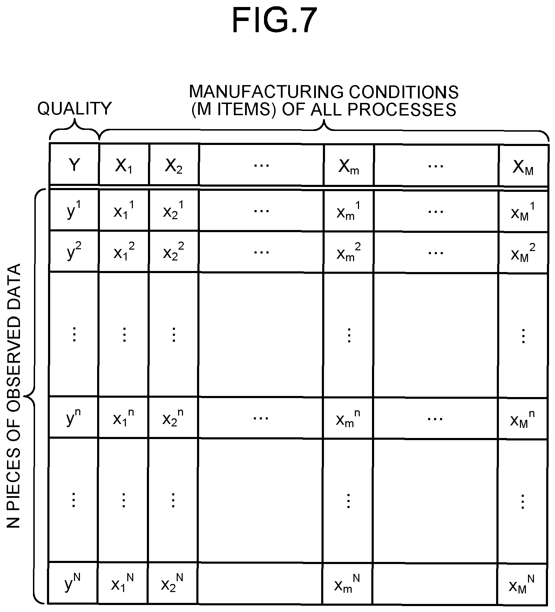

The integrated process record edition section 16 then identifies the position from which each metal material was taken, while taking into account the leading end portion and the trailing end portion truncated in each process, and, for the predetermined area of each metal material in the final process, associates the quality in the predetermined area with the manufacturing conditions of all processes in that predetermined area, and stores them in the record database 17. For example, in FIG. 6, the shaded area from which material A11 was taken in the final process, process 3, is identified back to material A1 in process 2 and material A in process 1. By repeating such processing for all metal materials, as illustrated in FIG. 7, record data on the manufacturing conditions (and quality) of metal materials in all processes are aligned and combined in units of the length of the metal materials. The following description returns back to FIG. 2 and continues.

The model generation section 18 generates a quality prediction model that predicts the quality of a metal material for each of predetermined areas based on the manufacturing condition for each of the predetermined areas in each process (step S4). Then, the prediction section 19 uses the quality prediction model generated by the model generation section 18 to predict the quality of a metal material manufactured under a certain manufacturing condition for each of predetermined areas (step S5).

According to the quality prediction model generation method, the quality prediction model, the quality prediction method, the quality prediction model generation device, and the quality prediction device according to the present embodiment as described above, by generating a quality prediction model that associates a manufacturing condition of each process with the quality of a metal material manufactured under the manufacturing condition for each of predetermined areas, the quality of a metal material with respect to a certain manufacturing condition can be predicted more accurately than it is conventionally.

In other words, the quality prediction model generation method, the quality prediction model, the quality prediction method, the quality prediction model generation device, and the quality prediction device according to the present embodiment combines the record data of the manufacturing condition (and quality) of all processes while aligning them in units of the length of the metal material at the output side of the final process, by taking into account, for each process, whether the leading end and the trailing end of the metal material have been interchanged, whether the front face and the back face of the metal material have been interchanged, and the cutting position and others. Thus, the record data of the manufacturing condition that are collected in detail in the longitudinal direction of the metal material by sensors, is effectively used to predict quality, so that the quality can be predicted with higher accuracy than it is conventionally.

When the quality prediction method according to the present embodiment is applied to a manufacturing method of a metal material, for example, the following processing is performed. First, a manufacturing condition confirmed during manufacturing of the metal material is fixed, and then the quality of the metal material manufactured under the fixed manufacturing condition is predicted for each of predetermined areas by the quality prediction method according to the present embodiment. Second, the manufacturing condition of a subsequent process is then changed based on the predicted results. The manufacturing condition is changed such that the quality of the manufactured metal material in every predetermined area included over the entire length of the metal material is within a predetermined control range. Thus, by applying the quality prediction method according to the present embodiment to a manufacturing method of a metal material, the final quality of the metal material can be predicted at a stage during manufacturing and a manufacturing condition can be changed accordingly, and thus the quality of the metal material to be manufactured is improved.

[Examples]

Examples of the quality prediction method according to the present embodiment will be explained. In an example, the quality prediction method according to the present embodiment was applied to the prediction of tensile strength of a highly workable high-strength cold-rolled steel sheet, which is a type of cold-rolled thin steel sheet.

The objective variable (quality) of the quality prediction in this example is the tensile strength of a product (highly workable high-strength cold-rolled steel sheet), and the explanatory variables (manufacturing conditions) are the chemical composition of the metal material in the smelting process, the temperature of the metal material in the casting process, the temperature of the metal material in the heating process, the temperature of the metal material in the hot rolling process, the temperature of the metal material in the cooling process, the temperature of the metal material in the cold rolling process, the temperature of the metal material in the annealing process, and others.

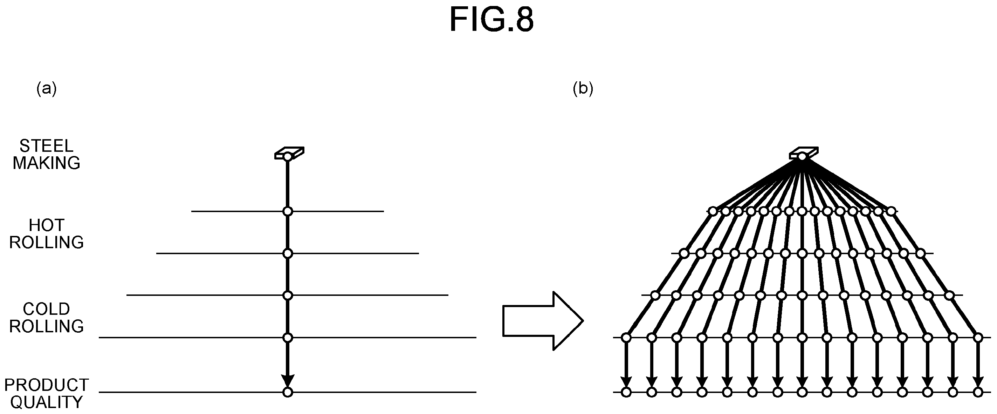

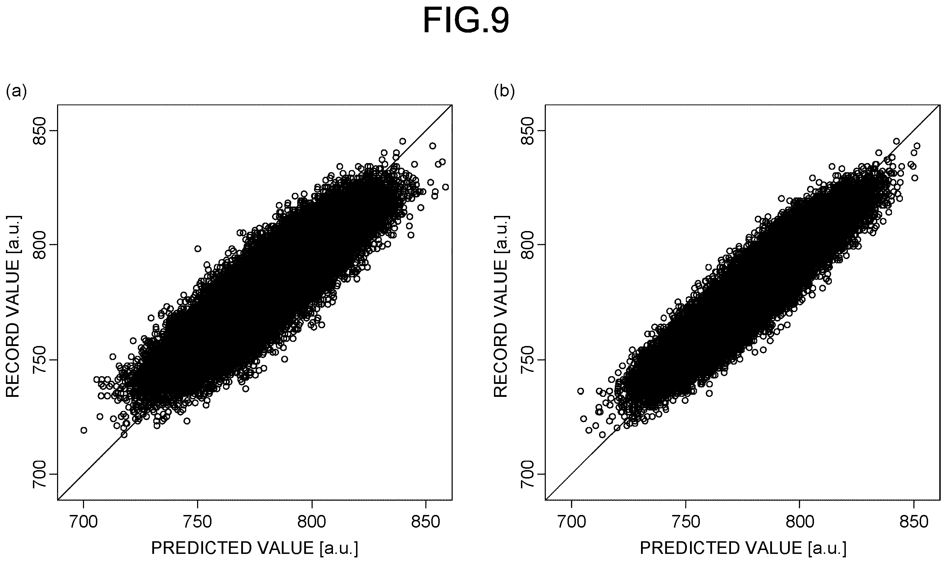

In this example, prediction results were compared between a case where prediction was made based on a conventional record database (refer to FIG. 8(a)) in which one representative value, such as an average value, for each manufacturing condition and each quality is stored for each product, and a case where prediction was made based on a quality prediction model generated from a record database (refer to FIG. 8(b)) of the quality prediction method according to the present embodiment. The number of samples in the record database was 40000, the number of explanatory variables was 45, and the prediction method used was local regression. As a record of the prediction, as illustrated in FIG. 9, the prediction error by the quality prediction method according to the present embodiment (refer to FIG. 9(b)) was confirmed that the root mean square error (RMSE) can be reduced by 23% compared to the prediction error by the conventional quality prediction method (refer to FIG. 9(a)).

The quality prediction method according to the present embodiment was also applied to the prediction of the front and back surface hardness of a thick steel sheet. The objective variable is the front and back surface hardness of a product, and the explanatory variables are the chemical composition in the smelting process, the front and back surface temperatures in the casting process, the front and back surface temperatures in the heating process, the front and back surface temperatures in the rolling process, the front and back surface temperatures in the cooling process, and others.

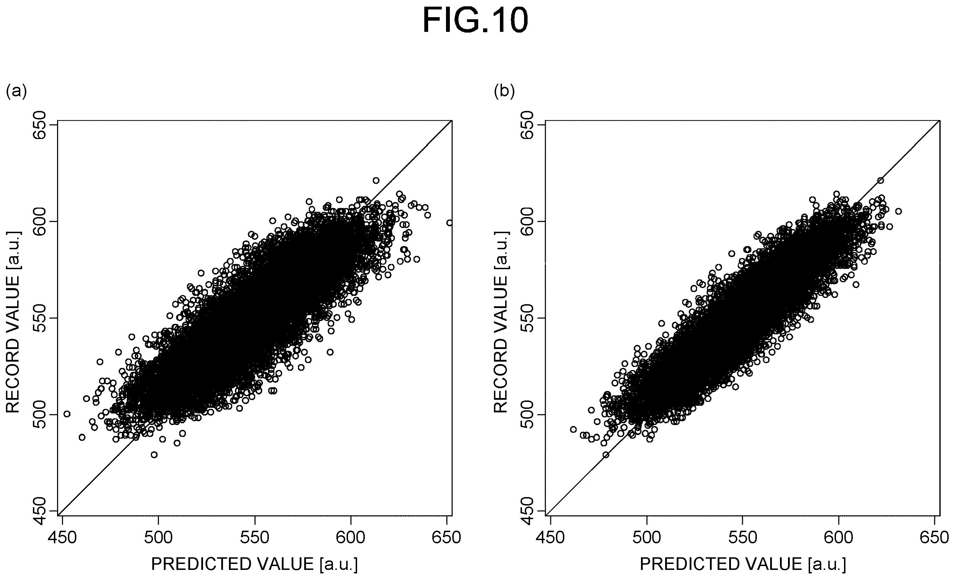

Prediction records were compared between a case where prediction was made based on a conventional record database (refer to FIG. 8(a)) in which one representative value, such as an average value, for each manufacturing condition and each quality is stored for each product, and a case where prediction was made based on a quality prediction model generated from a record database (refer to FIG. 8(b)) of the quality prediction method according to the present embodiment. The number of samples in the record database was 10000, the number of explanatory variables was 30, and the prediction method used was linear regression. As a record of the prediction, as illustrated in FIG. 10, the prediction error by the quality prediction method according to the present embodiment (refer to FIG. 10(b)) was confirmed that the RMSE can be reduced by 26% compared to the prediction error by the conventional quality prediction method (refer to FIG. 10(a)).

The quality prediction method according to the present embodiment was also applied to the prediction of front and back surface defects of a hot-dip galvanized steel sheet, which is a type of cold-rolled thin steel sheet. The objective variable was the presence or absence of the front and back surface defects on the product, and the explanatory variables are the chemical composition in the smelting process, the front and back surface temperatures, the meniscus flow rate, and the molten metal surface level in the casting process, the front and back surface temperatures in the heating process, the front and back surface temperatures in the hot rolling process, the front and back surface temperatures in the cooling process, the acid concentration and acid temperature in the pickling process, the front and back surface temperatures in the cold pressing process, the front and back surface temperatures in the annealing process, the amount of plating adhesion and the degree of alloying in the plating process, and others.

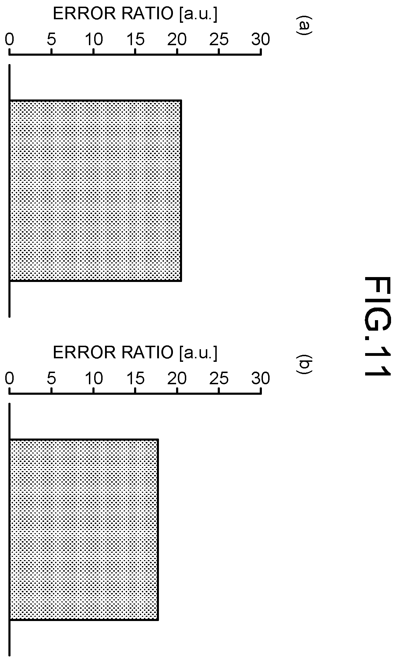

Prediction records were compared between a case where prediction was made based on a conventional record database (refer to FIG. 8(a)) in which one representative value, such as an average value, for each manufacturing condition and each quality is stored for each product, and a case where prediction was made based on a quality prediction model generated from a record database (refer to FIG. 8(b)) of the quality prediction method according to the present embodiment. The number of samples in the record database was 4,000, the number of explanatory variables was 250, and the prediction method used was decision trees. As a record of the prediction, as illustrated in FIG. 11, it was confirmed that the error ratio by the quality prediction method according to the present embodiment (refer to FIG. 11(b)) can be reduced by 14% compared to the error ratio by the conventional quality prediction method (refer to FIG. 11(a)).

The quality prediction method according to the present embodiment was also applied to the prediction of tensile strength of a high-strength cold-rolled steel sheet, which is a type of cold-rolled thin steel sheet, and a manufacturing condition of a subsequent process was changed based on the prediction records. The following describes an example of changing the post-annealing cooling temperature, which is a manufacturing condition for the final stage of the cold rolling process, at a stage in manufacturing where the record values of manufacturing conditions of the steel making process, the hot rolling process, and up to a stage previous to the final stage of the rolling process have been obtained.

On the basis of the record values of the manufacturing conditions of the steel making process, the hot rolling process, and up to the stage previous to the final stage of the cold rolling process, and a reference value of the post-annealing cooling temperature, which is a manufacturing condition for the final stage of the cold rolling process, the tensile strength values at positions in the total length of the product predicted using the quality prediction method according to the present embodiment are presented as follows:

$$\hat{y}^1, \hat{y}^2, \cdots \hat{y}^L$$

When the cooling temperature after the annealing temperature changes by $\Delta\mu$ from the reference value, the amounts of change in tensile strength at the positions in the total length of the product predicted using the quality prediction method according to the present embodiment are presented as follows:

$$\Delta\hat{y}^1(\Delta u), \Delta\hat{y}^2(\Delta u), \cdots \Delta\hat{y}^L(\Delta u)$$

On the basis of the above, the optimization problem expressed in the following equation (1) is solved:

$$\Delta u^* = arg \min_{\Delta u}|\Delta u| \cdot\cdot subject\ to \cdot\cdot$$
$$y_{LL} \leq \hat{y}^k + \Delta\hat{y}^k(\Delta u) \leq y_{UL}, (k = 1, 2, \cdots, L) \tag{1}$$

Here, in the above equation (1), $y_{LL}$ and $y_{UL}$ are the lower and upper control limits of tensile strength, respectively, and $\Delta\mu^*$ is the optimal solution of this optimization problem. This optimization problem can be solved by a mathematical programming method such as the branch-and-bound method. By changing the cooling temperature after the annealing temperature by $\Delta\mu^*$, it is possible to obtain a cold-rolled steel sheet in which the tensile strength of the entire length does not fall outside the control range, that is, there is no quality defect over the entire length.

As described above, the record data stored in the record database of the quality prediction method according to the present embodiment can be combined in a manner that hardness or defect presence can be retroactively combined with detailed manufacturing conditions of all processes, while taking into account the record data on whether the leading end and the trailing end have been interchanged, whether the front face and the back face have been interchanged, and the cutting position and others in each predetermined area of the metal material in the final process. Then, based on the quality prediction model generated from the record database so constructed, a predicted value under a certain manufacturing condition is calculated, making it possible to predict the quality of a metal material with high accuracy.

The quality prediction model generation method, the quality prediction model, the quality prediction method, the metal material manufacturing method, the quality prediction model generation device, and the quality prediction device according to the present invention have been described in detail in the descriptions of embodiments and examples. The present invention is, however, not limited to these descriptions, being defined by the appended claims. Various changes and modifications based on these descriptions can be considered, as long as they remain within the scope of the appended claims, which define the invention.

For example, in the aforementioned embodiment, the integrated process record edition section 16 identifies a predetermined area by taking into account, for every metal material in each process, whether the leading end and the trailing end have been interchanged, whether the front face and the back face have been interchanged, and the cutting position; however, not all of the processes of interchanging the leading and trailing ends of the metal material, interchanging the front and back faces of the metal material, and cutting the metal material are necessarily included in some cases. Therefore, the integrated process record edition section 16 may identify the predetermined area by taking into account at least one or more of the record data on: whether the leading end and the trailing end of the metal material have been interchanged; whether the front face and the back face of the metal material have been interchanged; and the cutting position of the metal material.

Reference Signs List

1 quality prediction device
11 manufacturing record collection section
12 manufacturing record edition section
13 leading-trailing end interchange record collection section
14 front-back face interchange record collection section
15 cutting record collection section
16 integrated process record edition section
17 record database
18 model generation section
19 prediction section

## Claims

1.   A metal material manufacturing method comprising:

fixing a manufacturing condition confirmed during manufacturing;
predicting quality of a metal material manufactured under the fixed manufacturing condition for each of predetermined areas by a quality prediction method using a quality prediction model generated by a quality prediction model generation method performed by a personal computer or a workstation for a metal material such as a steel slab or a steel sheet manufactured through one or more processes, the method comprising:

a first collection step of collecting a manufacturing condition of each of the processes for each of predetermined areas of the metal material, the predetermined areas being in the form of an area in the longitudinal direction of the steel slab or steel sheet, the values of the manufacturing conditions in the longitudinal direction of the metal material for each process include an actual measured value of a manufacturing condition measured by a sensor or a preset value of the manufacturing condition, and wherein each of the predetermined areas is determined based on a travel distance of the metal material in a conveyance direction in each process;
a second collection step of evaluating and collecting quality of the metal material manufactured through each process for each of the predetermined areas;
a storage step of storing the manufacturing condition of each process and the quality of the metal material manufactured under the manufacturing condition in association with each other for each of the predetermined areas;
a model generation step of generating a quality prediction model that predicts quality of the metal material for each of the predetermined areas based on the stored manufacturing condition for each of the predetermined areas in each process, and
wherein the model generation step generates the quality prediction model using machine learning including linear regression, local regression, principal component regression, PLS regression, a neural network, a regression tree, a random forest, and XGBoost; and
changing the manufacturing condition of a subsequent process based on a predicted result.

**2.** The metal material manufacturing method according to claim 1, comprising, before the storage step, a third collection step of collecting at least one or more of whether a leading end and a trailing end of the metal material have been interchanged in each process, whether a front face and a back face of the metal material have been interchanged in each process, and a cutting position of the metal material in each process, wherein the storage step identifies the predetermined areas by taking into account, on the metal material in each process, at least one or more of whether the leading end and the trailing end have been interchanged, whether the front face and the back face have been interchanged, and the cutting position, and stores the manufacturing condition of each process and the quality of the metal material produced under the manufacturing condition in association with each other for each of the predetermined areas.

**3.** The metal material manufacturing method according to claim 1, wherein

in a case where a shape of the metal material is deformed by going through each process,
the storage step identifies the predetermined area by evaluating a volume of the metal material from the leading end, and stores the manufacturing condition of each process and the quality of the metal material manufactured under the manufacturing condition in association with each other for each of the predetermined areas.

**4.** The metal material manufacturing method according to claim 1, wherein changing the manufacturing condition is performed such that the quality of the manufactured material in every predetermined area included over an entire length of the metal material is within a predetermined control range.

**Patentansprüche**

**1.** Verfahren zum Herstellen eines Metallwerkstoffs, umfassend:

Festlegen einer während des Herstellens bestätigten Herstellungsbedingung,
Vorhersagen der Qualität eines unter den festgelegten Herstellungsbedingung hergestellten Metallmaterials für jeden der vorbestimmten Bereiche mittels eines Qualitätsvorhersageverfahrens unter Verwendung eines Qualitätsvorhersagemodells, das durch ein von einem Arbeitsplatzrechner oder Bildschirm-Arbeitsplatz durchgeführtes Verfahren zur Erzeugung eines Qualitätsvorhersagemodells für ein Metallmaterial wie zum Beispiel eine Stahlbramme oder ein Stahlblech, das durch einen oder mehrere Prozesse hergestellt wird, erzeugt wurde, wobei das Verfahren umfasst:

einen ersten Erfassungsschritt zum Erfassen einer Herstellungsbedingung jedes der Prozesse für jeden der vorbestimmten Bereiche des Metallmaterials, wobei die vorbestimmten Bereiche die Form eines Bereichs in Längsrichtung der Stahlbramme oder des Stahlblechs haben, die Werte der Herstellungsbedingungen in Längsrichtung des Metallmaterials für jeden Prozess einen tatsächlichen Messwert einer durch einen Sensor gemessenen Herstellungsbedingung oder einen voreingestellten Wert der Herstellungsbedingung beinhalten und wobei jeder der vorbestimmten Bereiche auf der Grundlage einer zurückgelegten Strecke des Metallmaterials in einer Förderrichtung in jedem Prozess bestimmt wird,
einen zweiten Erfassungsschritt zum Bewerten und Erfassen der Qualität des durch jeden Prozess hergestellten Metallmaterials für jeden der vorbestimmten Bereiche,
einen Speicherschritt zum Speichern der Herstellungsbedingung jedes Prozesses und der Qualität des unter der Herstellungsbedingung hergestellten Metallmaterials miteinander verbunden für jeden der vorbestimmten Bereiche,
einen Modellgenerierungsschritt zum Erzeugen eines Qualitätsvorhersagemodells, das die Qualität des Metallmaterials für jeden der vorbestimmten Bereiche auf der Grundlage der gespeicherten Herstellungsbedingung für jeden der vorbestimmten Bereiche in jedem Prozess vorhersagt, und
wobei der Modellgenerierungsschritt das Qualitätsvorhersagemodell unter Verwendung von maschinellem Lernen erzeugt, einschließlich linearer Regression, lokaler Regression, Hauptkomponentenregression, PLS-Regression, eines neuronalen Netzwerks, eines Regressionsbaums, eines Random Forests und XGBoost, und
Ändern der Herstellungsbedingung eines nachfolgenden Prozesses auf der Grundlage eines vorhergesagten Ergebnisses.

**2.** Verfahren zum Herstellen eines Metallwerkstoffs gemäß Anspruch 1, umfassend, vor dem Speicherschritt, einen dritten Erfassungsschritt zum Erfassen mindestens eines oder mehrerer von: ob in jedem Prozess ein vorderes Ende und ein hinteres Ende des Metallmaterials vertauscht wurden, ob in jedem Prozess eine Vorderseite und eine Rückseite des Metallmaterials vertauscht wurden, und eine Schnittposition des Metallmaterials in jedem Prozess, wobei

der Speicherschritt die vorbestimmten Bereiche identifiziert, indem er beim Metallmaterial in jedem Prozess mindestens eines oder mehrere berücksichtigt von: ob das vordere Ende und das hintere Ende vertauscht wurden, ob die Vorderseite und die Rückseite vertauscht wurden, und die Schnittposition, und die Herstellungsbedingung jedes Prozesses und die Qualität des unter der Herstellungsbedingung hergestellten Metallmaterials für jeden der vorbestimmten Bereiche miteinander verbunden speichert.

3. Verfahren zum Herstellen eines Metallwerkstoffs gemäß Anspruch 1, wobei

in einem Fall, in dem eine Form des Metallmaterials durch Durchlaufen jedes Prozesses verformt wird, der Speicherschritt den vorbestimmten Bereich identifiziert, indem er ein Volumen des Metallmaterials vom vorderen Ende aus bewertet und die Herstellungsbedingung jedes Prozesses und die Qualität des unter der Herstellungsbedingung hergestellten Metallmaterials für jeden der vorbestimmten Bereiche miteinander verbunden speichert.

4. Verfahren zum Herstellen eines Metallwerkstoffs gemäß Anspruch 1, wobei die Änderung der Herstellungsbedingung so durchgeführt wird, dass die Qualität des hergestellten Materials in jedem vorbestimmten Bereich, der über eine gesamte Länge des Metallmaterials enthalten ist, innerhalb eines vorbestimmten Kontrollbereichs liegt.

## Revendications

1. Procédé de fabrication d'un matériau métallique comprenant :

la fixation d'une condition de fabrication confirmée pendant la fabrication ;
la prédiction, pour chacune de zones prédéterminées, d'une qualité d'un matériau métallique fabriqué sous la condition de fabrication fixée, par un procédé de prédiction de la qualité utilisant un modèle de prédiction de qualité généré par un procédé de génération de modèle de prédiction de la qualité effectué par un ordinateur personnel ou un poste de travail, pour un matériau métallique tel qu'une brame d'acier ou une tôle d'acier fabriquée par le biais d'un ou plusieurs processus, le procédé comprenant : une première étape de collecte consistant à collecter, pour chacune de zones prédéterminées du matériau métallique, une condition de fabrication de chacun des processus, les zones prédéterminées étant sous la forme d'une zone dans la direction longitudinale de la brame d'acier ou de la tôle d'acier, les valeurs des conditions de fabrication dans la direction longitudinale du matériau métallique pour chaque processus comportent une valeur mesurée réelle d'une condition de fabrication mesurée par un capteur ou une valeur prédéfinie de la condition de fabrication, et dans lequel chacune des zones prédéterminées est déterminée sur la base d'une distance de déplacement du matériau métallique dans une direction de convoyage lors de chaque processus ;
une deuxième étape de collecte consistant à évaluer et à collecter, pour chacune des zones prédéterminées, une qualité du matériau métallique fabriqué par le biais de chaque processus ;
une étape de stockage consistant à stocker, en association l'une avec l'autre pour chacune des zones prédéterminées, la condition de fabrication de chaque processus et la qualité du matériau métallique fabriqué sous ladite condition de fabrication ;
une étape de génération de modèle consistant à générer, sur la base de la condition de fabrication stockée pour chacune des zones prédéterminées lors de chaque processus, un modèle de prédiction de qualité qui prédit une qualité du matériau métallique pour chacune des zones prédéterminées, et
dans lequel l'étape de génération de modèle génère le modèle de prédiction de qualité au moyen d'un apprentissage automatique comportant une régression linéaire, une régression locale, une régression en composantes principales, une régression PLS, un réseau neuronal, un arbre de régression, une forêt aléatoire, et XGBoost ; et
changer la condition de fabrication d'un processus subséquent sur la base d'un résultat prédit.

2. Procédé de fabrication d'un matériau métallique selon la revendication 1, comprenant, avant l'étape de stockage, une troisième étape de collecte consistant à collecter au moins une ou plusieurs parmi l'information indiquant si une extrémité d'attaque et une extrémité de fuite du matériau métallique ont été interverties lors de chaque processus, l'information indiquant si une face avant et une face arrière du matériau métallique ont été interverties lors de chaque processus, et une position de coupe du matériau métallique lors de chaque processus, dans lequel

l'étape de stockage identifie les zones prédéterminées en tenant compte, sur le matériau métallique lors de chaque processus, d'au moins une ou plusieurs parmi l'information indiquant si l'extrémité d'attaque et l'extrémité de fuite ont été interverties, l'information indiquant si la face avant et la face arrière ont été interverties, et la position de coupe et

stocke, en association l'une avec l'autre pour chacune des zones prédéterminées, la condition de fabrication de chaque processus et la qualité du matériau métallique produit sous ladite condition de fabrication.

3. Procédé de fabrication d'un matériau métallique selon la revendication 1, dans lequel

dans le cas où une forme du matériau métallique est déformée lors du passage à travers chaque processus, l'étape de stockage identifie la zone prédéterminée en évaluant un volume du matériau métallique à partir de l'extrémité d'attaque, et stocke, en association l'une avec l'autre pour chacune des zones prédéterminées, la condition de fabrication de chaque processus et la qualité du matériau métallique fabriqué sous la condition de fabrication.

4. Procédé de fabrication d'un matériau métallique selon la revendication 1, dans lequel le changement de la condition de fabrication est effectué de sorte que la qualité du matériau fabriqué dans chaque zone prédéterminée comprise sur toute la longueur du matériau métallique se situe dans une plage de commande prédéterminée.

# FIG.1

# FIG.2

START

**S1**
COLLECT RECORD DATA ON MANUFACTURING CONDITION AND QUALITY FOR EACH PROCESS

**S2**
COLLECT, FOR METAL MATERIAL, RECORD DATA ON WHETHER LEADING END AND TRAILING END HAVE BEEN INTERCHANGED, WHETHER FRONT FACE AND BACK FACE HAVE BEEN INTERCHANGED, CUTTING POSITION AND OTHERS

**S3**
CONVERT RECORD DATA INTO DATA IN UNITS OF LENGTH OF METAL MATERIAL

**S4**
ALIGN AND COMBINE RECORD DATA OF ALL PROCESSES IN UNITS OF LENGTH OF METAL MATERIAL

**S5**
GENERATE QUALITY PREDICTION MODEL

**S6**
PREDICT QUALITY OF METAL MATERIAL USING QUALITY PREDICTION MODEL

END

# FIG.3

RESULT VALUES OF PLURALITY OF
MANUFACTURING CONDITIONS
(AND QUALITY)

| | TIME | SPEED | MANUFAC-TURING CONDITION 1 | MANUFAC-TURING CONDITION 2 | ... |
|---|---|---|---|---|---|
| | $t^1$ | $v^1$ | $x_1{}^1$ | $x_2{}^1$ | ... |
| | $t^2$ | $v^2$ | $x_1{}^2$ | $x_2{}^2$ | ... |
| | $\vdots$ | $\vdots$ | $\vdots$ | $\vdots$ | |

PLURALITY OF PIECES OF OBSERVED DATA IN FIXED CYCLE (UNITS OF TIME)

# FIG.4

RESULT VALUES OF PLURALITY OF
MANUFACTURING CONDITIONS
(AND QUALITY)

| | LENGTH | MANUFAC-TURING CONDITION 1 | MANUFAC-TURING CONDITION 2 | ⋯ |
|---|---|---|---|---|
| | $l^1$ | $x_1^{\ 1}$ | $x_2^{\ 1}$ | ⋯ |
| | $l^2$ | $x_1^{\ 2}$ | $x_2^{\ 2}$ | ⋯ |
| | ⋮ | ⋮ | ⋮ | |

PLURALITY OF PIECES OF OBSERVED DATA IN FIXED CYCLE (UNITS OF LENGTH)

# FIG.5

PROCESS 1    PROCESS 2    PROCESS 3

MATERIAL A — MATERIAL A1 — MATERIAL A11  ⌐--·B

MATERIAL A12

MATERIAL A2 — MATERIAL A21

# FIG.6

PROCESS 1

250mm (LEADING END PORTION)

3050mm

MATERIAL A 1650mm

350mm (TRAILING END PORTION)

COLLECT EVERY 50mm

5300mm

INTERCHANGE LEADING END AND TRAILING END

PROCESS 2

500mm (LEADING END PORTION)

34000mm

32300mm

1200mm (TRAILING END PORTION)

MATERIAL A1

COLLECT EVERY 100mm

68000mm

PROCESS 3

2500mm (LEADING END PORTION)

5300mm (TRAILING END PORTION)

57200mm

MATERIAL A11

COLLECT EVERY 500mm

65000mm

EP 4 006 670 B1

# FIG.7

| QUALITY | MANUFACTURING CONDITIONS (M ITEMS) OF ALL PROCESSES | | | | | |
|---|---|---|---|---|---|---|
| $Y$ | $X_1$ | $X_2$ | $\cdots$ | $X_m$ | $\cdots$ | $X_M$ |
| $y^1$ | $x_1{}^1$ | $x_2{}^1$ | $\cdots$ | $x_m{}^1$ | $\cdots$ | $x_M{}^1$ |
| $y^2$ | $x_1{}^2$ | $x_2{}^2$ | $\cdots$ | $x_m{}^2$ | $\cdots$ | $x_M{}^2$ |
| $\vdots$ | $\vdots$ | $\vdots$ | | $\vdots$ | | $\vdots$ |
| $y^n$ | $x_1{}^n$ | $x_2{}^n$ | $\cdots$ | $x_m{}^n$ | $\cdots$ | $x_M{}^n$ |
| $\vdots$ | $\vdots$ | $\vdots$ | | $\vdots$ | | $\vdots$ |
| $y^N$ | $x_1{}^N$ | $x_2{}^N$ | | $x_m{}^N$ | | $x_M{}^N$ |

N PIECES OF OBSERVED DATA

# FIG.8

(a)

(b)

STEEL
MAKING

HOT
ROLLING

COLD
ROLLING

PRODUCT
QUALITY

# FIG.9

(a)

(b)

FIG.10

# FIG.11

(a)

(b)

**EP 4 006 670 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019074969 A
- JP 2004355189 A
- JP 2006309709 A
- JP 2008112288 A
- JP 2009230412 A
- JP 2014013560 A
- JP 2014071858 A
- JP 2014071859 A
- JP 2017120638 A